# DEMANDE DE BREVET EUROPEEN

(11) **EP 1 953 234 A1**
(43) Date de publication de la demande: **06.08.2008**
(21) Numéro de dépôt: 07101464.1
(22) Date de dépôt: 31.01.2007
(51) Int. Cl.: C12P 7/56

(54) **Procédé de production d'acide lactique par fermentation d'un milieu autosuffisant à base de jus vert de canne**

(71) Demandeur: Galactic S.A., 7760 Escanaffles (BE)
(72) Inventeur: Coszach, Philippe, 7760, Escanaffles (BE); Bogaert, Jean-Christophe, 7760, Escanaffles (BE); Mariage, Pierre-Antoine, 7760, Escanaffles (BE)
(74) Mandataire: Vandeberg, Marie-Paule L.G.

(57) **Abrégé**

L'invention concerne un procédé production d'acide lactique par fermentation d'un extrait de canne à sucre, au moyen de micro-organisme appartenant aux genres Bacillus ou Sporolactobacillus. Le milieu de fermentation est autosuffisant.

Le procédé peut comporter au moins une étape de purification. L'acide lactique produit est stable à la chaleur et d'une pureté particulièrement élevée.

## Description

### Domaine de l'invention

L'acide lactique ou acide hydroxy-2-propanoïque est un acide carboxylique α-hydroxylé qui peut être produit par fermentation. D'autres voies d'obtention de l'acide lactique sont connues de l'homme de l'art, via des transformations chimiques de réactifs issus de la pétrochimie telles que l'hydrolyse du lactonitrile lui-même obtenu au départ d'acétaldéhyde, la chloration et l'hydrolyse d'acide propionique ou encore via la nitration du propène.

L'acide lactique existe sous deux formes diastéréoisomérique : acide L(+) et D(-) lactique et rencontre chaque jour de nouvelles applications, depuis l'utilisation classique en tant que conservateur alimentaire jusqu'à de nouveaux développements tels que la synthèse de solvants, pesticides, herbicides, polymères biodégradables, ...

Cependant, en raison du renforcement croissant des critères de qualité requis et la nécessité d'atteindre des coûts de production compatibles avec le marché des commodités, il est crucial de pouvoir réduire les coûts des matières premières tout en assurant une qualité compatible avec les applications les plus exigeantes.

### Etat de l'art

La pureté d'un acide lactique est entre autre évaluée par un test de stabilité thermique consistant à mesurer la coloration (échelle APHA en unité Hazen) du produit après chauffe à reflux à 200°C pendant 2h. Si l'acide lactique refroidi à température ambiante après ce test a une coloration inférieure à 50 Hazen, il sera considéré comme stable à la chaleur. Il n'est cependant pas rare de rencontrer sur le marché des applications particulières nécessitant une coloration après chauffe inférieure à 20 Hazen, voire à 10 Hazen.

L'acide lactique, par exemple, servant de matière première pour la production d'acide polylactique doit présenter une pureté très élevée et un indice de thermostabilité très bas de l'ordre de 0 -50 Hazen et préférentiellement de 0 - 20 Hazen. Il correspond à un grade « polymère ».

La production d'acide lactique par fermentation peut être réalisée en ajoutant un micro-organisme producteur d'acide lactique à un milieu contenant une source de carbone fermentescible purifié, des sels minéraux (source d'azote, de phosphate, de soufre et d'oligoéléments tels que le zinc, le magnésium, le manganèse,...) et une source d'azote organique composée d'acides aminés libres ou liés sous forme d'oligopeptides et de peptides, de vitamines et de traces de co-facteurs enzymatiques. Il est également connu que les micro-organismes habituellement utilisés des genres Lactobacillus, Bacillus et Sporolactobacillus ne peuvent croître et produire de l'acide lactique de manière industrielle sans l'ajout d'une telle source d'azote organique, que sa composition soit définie ou qu'il s'agisse d'un extrait naturel.

Des exemples de substrats carbonés respectant ces spécifications sont les sucres purifiés de betteraves, de canne et les sirops de glucose raffinés provenant de l'hydrolyse de l'amidon de maïs, blé, pomme de terre et autres.

Des exemples de sources d'azote organique respectant les spécifications citées plus haut sont les autolysats et hydrolysats de levure, les hydrolysats de protéines végétales (tryptone de soja, peptone de gluten,...) et animales (peptone de caséine,...) et également les sous-produits solubles de trempe de blé et de maïs. Ces sources d'azote organique sont vendues à des prix relativement élevés, ce qui grève fortement le coût de fabrication de l'acide lactique. Des sources d'azote organique définies, reconstituées à partir d'acides aminés, de vitamines et de promoteurs de croissance purifiés peuvent également être utilisées mais avec des coûts encore supérieurs.

Pour diminuer les coûts de production, l'acide lactique par fermentation peut également être obtenue en ajoutant un micro-organisme à une source de sucres fermentescibles, bon marché, non-raffinés, constituée d'intermédiaires ou de sous-produits de l'industrie agricole (sirop d'amidon, lactosérum, jus vert de canne, mélasse, bagasse de canne hydrolysé,...) additionné d'azote organique tel que des sous-produits solubles de trempe de blé et/ou des extraits de levures.

Des impuretés organiques sont présentes en quantités importantes dans ces sources de sucres non raffinés. Cette quantité d'impuretés organiques (hors glucides) peut être mesurée en analysant la quantité d'azote organique d'un échantillon Celle-ci est exprimée en g/kg et est mesurée par la méthode Kjeldhal.

Ces impuretés organiques peuvent être d'origine végétale ou encore être le résultat de réactions de caramélisation ou de réactions de Maillard intervenant lors des différentes étapes de traitement du végétal. Elles peuvent être des alcools, des acides organiques, des aldéhydes, des produits de dégradation des sucres (furanes, pyrones, cyclopentènes, acides organiques, aldéhydes, composés sulfurés, pyrroles, pyridines, imidazoles, pyrazines, oxazoles, thiazoles,...), des protéines et des vitamines.

Néanmoins, dans ces sources de sucres non-raffinés, quelques unes des impuretés, tel que les furfurals et les hydroxyméthylfurfurals, sont connues pour inhiber la croissance de certains micro-organismes et ralentir les procédés de bioconversion comme la fermentation lactique. Ainsi Payot et al. (1998) ont montré que la mélasse de betterave contenait des composés inhibiteurs de croissance pour Bacillus Coagulans.

Il est fait mention de l'utilisation d'un milieu à base de jus vert de canne et des extraits de levures (3 g/l) pour la production d'acide lactique par fermentation à partir de levures de vin génétiquement modifiées. Cependant, ces levures ne sont pas homofermentaires et ont un rendement carboné (acide lactique produit sur sucre consommé) de 61%.

La purification industrielle d'un acide lactique de grade thermostable à partir d'un jus de fermentation riche en acide lactique peut être réalisée par différentes technologies qui comprennent en générale des étapes communes :
- clarification du moût de fermentation : (centrifugation, floculation/filtration, microfiltration,...) ;
- élimination des ions (électrodialyse, résines, extraction liquide/liquide,...) ;
- élimination de la couleur et autres impuretés (nano-filtration, charbon actif,...) ;
- concentration/distillation de l'acide lactique, ces étapes devant être couplées de manière à obtenir un rendement élevé ;
- d'autres techniques peuvent également être utilisées comme la cristallisation.

Certaines des impuretés présentes dans les sources de sucres non raffinées ont des poids moléculaires et des courbes de tensions de vapeurs proches de celle de l'acide lactique (tel que le 5-hydroxyméthylfurfural), ce qui les rend difficile à séparer de l'acide lactique. D'autres sont des acides organiques et ne pourront être séparés efficacement de l'acide lactique par des procédés classiques tel que par exemple l'électrodialyse, la cristallisation ou encore l'extraction liquide-liquide.

Il est ainsi reconnu que la production industrielle d'acide lactique stable à la chaleur- et spécialement de grade « polymère » doit être réalisée à partir d'un milieu de fermentation contenant une faible teneur en impuretés. La présence d'impuretés organiques tels que des acides organiques, des aldéhydes et des alcools dans le jus de fermentation rend l'acide lactique difficile et cher à purifier.

Un exemple de la difficulté de produire un acide lactique de qualité stable à la chaleur à partir d'une source de substrat carboné non raffiné est donné par le document WO 2006/001034 A2. Ce document décrit la production d'acide polylactique par fermentation de sources de carbone bon marché, issues de l'agriculture comme la mélasse de canne par des souches du genre Lactobacillus. Afin d'obtenir une croissance et une productivité suffisante, une source d'azote organique exogène (sous-produits solubles de trempe de maïs et pâte de levure) est ajoutée au milieu. Le procédé décrit clairement que l'acide lactique purifié obtenu à partir de mélasse de canne à sucre est de couleur brune. La purification est réalisée après formation du dimère cyclique de l'acide lactique (le lactide) à partir d'une solution impure d'acide lactique concentrée. Ce qui a pour conséquence la formation de nombreux sous-produits provenant des réactions des impuretés entre elles et avec l'acide lactique. Pour purifier ce lactide, le document décrit une recristallisation en solvant à base d'acétate d'éthyle et ne mentionnent pas de rendement de récupération de lactide purifié. Or, il est connu de l'homme de l'art que le lactide sous ses formes L, D et méso est soluble partiellement dans l'acétate d'éthyle, entraînant une perte importante de produit et des mauvais rendements de production.

Lors de la production de sucre à partir de la canne, le sucre est d'abord extrait de la canne par extraction mécanique ou diffusion. Le jus extrait de cette étape est appelé ci-dessous « jus vert de canne ». Ce jus vert de canne est ensuite carbonaté et filtré pour extraire les impuretés non-solubles et les anions organiques. Le filtrat est enfin concentré par évaporation et donne un sirop de sucre qui est appelé ci-dessous « sirop de canne brut ». Ce sirop peut être inversé (hydrolyse du saccharose en glucose et fructose) pour donner du « sirop de canne brut inversé » ou cristallisé directement et donner ce qui est appelé ci-dessous du « sucre de canne brut » ou être purifié et cristallisé pour donner d'une part le « sucre de canne raffiné » et un sous-produit chargé en impuretés « la mélasse de canne ».

Le sucre peut être produit à partir de la betterave par un procédé similaire. Le sucre est d'abord extrait de la betterave par extraction mécanique ou diffusion. Le jus extrait de cette étape est appelé ci-dessous « jus de diffusion de betterave ». Ce jus est alors carbonaté et filtré pour extraire les impuretés non-solubles et les anions organiques. Le filtrat est alors concentré par évaporation et donne un sirop de sucre qui est appelé ci-dessous « sirop de betterave brut ». Ce sirop peut être cristallisé directement et donner ce qui est appelé ci-dessous du « sucre de betterave brut » ou être purifié et cristallisé pour donner d'une part le « sucre de betterave raffiné » et un sous-produit chargé en impuretés « la mélasse de betterave ».

Ainsi, jusqu'à présent, il était admis par l'homme de l'art que la production industrielle d'un acide lactique stable à la chaleur à partir de sucre nécessitait l'utilisation d'un milieu préparé à partir de sucre purifié, d'un substrat organique azoté et de minéraux.

### Brève description de l'invention

De tout ce qui précède, il résulte que jusqu'à présent la production d'acide lactique de grade stable à la chaleur répondant aux critères de qualité du marché (alimentaire, pharmaceutique, cosmétique) nécessite de réaliser une fermentation lactique sur un milieu :
- de composition et préparation complexe comprenant de nombreux ingrédients à mélanger dans des quantités très précises et nécessitant l'intervention d'un préparateur qualifié ;
- de prix généralement élevé (sucre purifié, source d'azote organique riche, nutriments minéraux purs).

Soucieuse de développer un procédé qui permette de répondre mieux aux contraintes pratiques et économiques, la demanderesse a constaté de manière surprenante que cet objectif pouvait être atteint par un procédé consistant en une fermentation lactique par des microorganismes du genre Bacilles et/ou Sporolactobacilles, d'un milieu autosuffisant préparé à partir de jus vert de canne sans ajout d'autres nutriments organiques et inorganiques, ou encore à partir d'un milieu composé de dérivés de jus vert de canne, riche en matières organiques azotées (comme le sirop brut de canne) sans ajout d'autres nutriments organiques.

Un milieu de fermentation qui comprend, comme source de glucides, un extrait végétal ou un dérivé d'extrait végétal est considéré comme autosuffisant pour la fermentation d'un micro-organisme, s'il permet à celui-ci de croître et produire ses métabolites sans ajout d'autres nutriments organiques que ceux présents dans l'extrait végétal ou du dérivé d'extrait végétal utilisé comme source de glucides.

L'acide lactique produit par fermentation peut alors être purifiée par les techniques décrites dans l'état de l'art (concentration, distillation, cristallisation, échange d'ions,...) afin de produire un acide lactique stable à la chaleur.

### Description détaillée de l'invention

Après de longues recherches sur de nombreux substrats carbonés et de nombreuses souches microbiennes productrices d'acide lactique, la demanderesse a découvert de manière surprenante que le jus vert de canne et ses dérivés (sirop de canne brut, sirop de canne brut inversé, jus vert de canne concentré, jus vert de canne sec, ...) :
- contenaient naturellement les sucres et les promoteurs organiques de croissance et de productivité nécessaires à certains micro-organismes industriels appartenant aux genres Bacillus et/ou Sporolactobacillus pour la fermentation lactique ;
- ne contenaient pas de composés capables d'inhiber la croissance des micro-organismes des genres Bacillus et/ou Sporolactobacillus ;
- ne contenaient pas d'impuretés empêchant la production d'un acide lactique de haute pureté par les procédés de purification et plus particulièrement ceux comportant une évaporation et/ou une distillation et/ou cristallisation et/ou échange d'ions ;
- que le jus vert de canne contenait également les sels minéraux nécessaires à la croissance du micro-organisme.

Ces propriétés inattendues du jus vert de canne et ses dérivés contenant des matières organiques azotées (sirop de canne brut, sirop de canne brut inversé, jus vert de canne concentré, jus vert de canne sec, ...) sont exploitées dans cette invention qui décrit un procédé original de production d'un acide lactique stable à la chaleur à partir de ces milieux.

Ces découvertes sont surprenantes étant donné que nos recherches ont également montré que les dérivés non raffinés de la betterave n'étaient pas autosuffisants et donc nécessitaient l'ajout d'un substrat azoté organique pour que certains micro-organismes appartenant aux genres Bacillus et Sporolactobacillus pour la fermentation lactique puissent atteindre des productivités équivalentes à celles observées en milieu industriel classique. Ces observations sont décrites dans les exemples.

De plus, comme également décrit dans les exemples, le jus vert de canne et ses dérivés contenant des matières organiques azotées ne constituent pas des milieux autosuffisants pour tous les micro-organismes producteurs d'acide lactique.

Ces découvertes sont d'autant plus surprenantes qu'en plus d'être un milieu autosuffisant pour certains microorganismes des genres Bacillus et/ou Sporolactobacillus, le milieu de fin de fermentation peut être purifié par les techniques classique d'évaporation, de distillation, de cristallisation et/ou échange d'ions, en un acide lactique stable à la chaleur et de grade « polymère » répondant aux critères de qualité du marché (alimentaire, pharmaceutique, cosmétique, industriel). Ce qui n'est par exemple pas le cas pour la mélasse de canne à sucre.

L'originalité dont a fait part la demanderesse réside dans le choix de cette matière première comme source à la fois de sucre bon marché, de minéraux et d'azote organique contrairement à d'autres recherches effectuées sur des sucres bon marché comme la mélasse auxquels on ajoute une source d'azote organique exogène comme des extraits de levures.

L'utilisation du jus vert de canne brut ou purifié, liquide ou solide comme milieu de fermentation autosuffisant comme décrit ci-dessus permet de réduire drastiquement les coûts de production de l'acide lactique et facilite la préparation des milieux de fermentation.

L'utilisation de jus vert de canne brut ou clarifié permet d'éviter l'utilisation d'énergie pour évaporer l'eau lors de sa concentration ou sa cristallisation, et ainsi réduire de manière importante la consommation d'énergie et la pollution environnementale.

Le procédé permet également d'éviter les pertes en glucides dues à la purification du sucre de canne (principalement mélasse). Le rendement global de production d'acide lactique à partir de la canne à sucre est ainsi supérieur. En effet, 1 tonne de canne à sucre contient 150 à 180 kg de sucre mais les procédés actuels de production ne permettent de produire qu'environ 120 kg de sucre de canne purifié soit environ 120 kg d'acide lactique après fermentation. L'utilisation directe du jus vert de canne ou de ses dérivés permettra l'utilisation de la quasi totalité du sucre présent dans la canne à sucre et donc de produire entre 150 et 180 kg d'acide lactique après fermentation, soit une augmentation de la quantité d'acide lactique produite de 20 à 30 %.

Le procédé permet également d'éliminer l'étape de clarification du jus vert de canne. Cette étape consiste à précipiter les impuretés organiques par de la chaux en formant des sels insolubles de calcium. Après de nombreuses recherches, nous avons découvert que l'étape de séparation de la biomasse soit par floculation, par microfiltration, par nano-filtration ou par toute autre technique, rendait inutile la clarification du jus vert de canne. Ceci simplifie fortement le procédé global de production d'acide lactique à partir de canne à sucre.

D'autres détails et particularités de l'invention, donnés ci-après à titre d'exemples non-limitatifs, ressortent de la description comme quelques formes possibles de sa réalisation.

### Exemple 1 : Production d'un jus vert sec de canne

Le jus vert sec de canne peut être produit par exemple par la technique suivante. Le jus vert de canne est extrait de la canne par extraction mécanique dans un moulin. Le jus est ensuite filtré à 10 µm pour extraire les impuretés non-solubles.

La composition du jus vert obtenu est donnée dans le tableau 1.

**Tableau 1: Composition du jus vert de canne**

| | **Teneur en moyenne en g/l** |
|---|---|
| **Saccharose** | 160 |
| **Glucose** | 5,7 |
| **Fructose** | 5,7 |
| **Protéines** | 0,95 |
| **Amidon** | 0,12 |
| **Gommes** | 1 |
| **Cires** | 0,24 |
| **K2O** | 1 |
| **CaO** | 0,4 |
| **MgO** | 0,53 |
| **P2O5** | 0,76 |
| **SiO2** | 6,7 |
| **SO3** | 1 |
| **Acides organiques (aconitique, citrique,...)** | 5,7 |
| **Divers (fibres, colloïdes, chlorophylle, polyphénols,...)** | 30 |

Le filtrat (18% de matière sèche) est alors concentré dans un évaporateur à 70°C et sous 700 mbars pour atteindre 85% de matière sèche.

Le concentré de jus vert de canne peut être envoyé à chaud vers un atomiseur à 150°C sous 125 mbars ou être mélangé avec 5% jus vert sec de canne (comme support de nucléation) et envoyé à chaud vers un tambour-sécheur à 150°C sous 125 mbars pour donner un produit sec.

### Exemple 2 : Culture de Bacillus Coagulans sur diverses sources de sucre

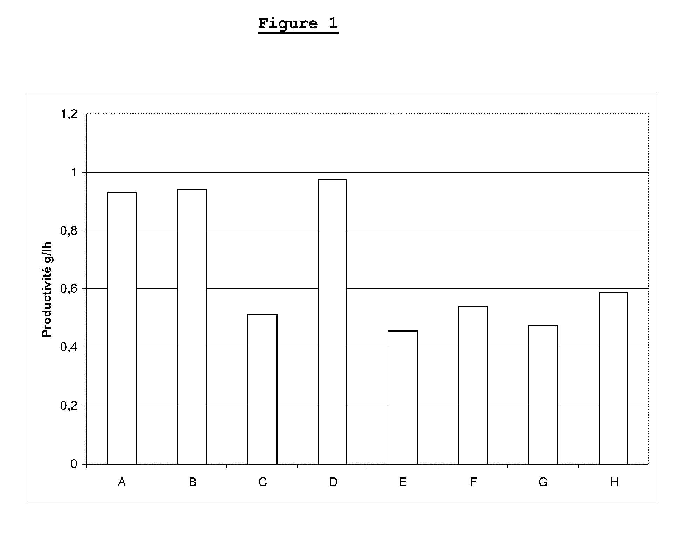

Une culture de Bacillus Coagulans (LMG 17452), a été cultivée dans des réacteurs BBraun 21 Biostat B sur un des milieux de fermentation décrit au tableau 2, à 52°C et maintenu à pH 6,2 avec du lait de Ca(OH)₂ 25% en poids. La culture a été maintenue en routine en transférant 250 ml de la culture toutes les 24 h dans un nouveau fermenteur contenant 750 ml de milieu. La vitesse de production d'acide lactique ou productivité en gramme par litre et par heure, a été suivie durant 5 fermentations d'affilée sur 8 milieux de compositions différentes (sur la figure 1, chaque colonne est représentative de la moyenne des résultats des 5 essais).

Sur la figure 1, nous pouvons clairement observer que les fermentations réalisées sur les milieux B (jus vert de canne sec sans sels et sans Yeast Extract ou extrait de levure) et D (sirop brut de canne sans Yeast Extract mais avec sels) ont des productivités d'acide lactique semblables aux fermentations sur le milieu A (sucre blanc avec Yeast Extract et sels).

De plus, les résultats montrent sans ambigüités que les fermentations sur les milieux C (sirop brut de canne sans Yeast Extract et sans sels) ainsi que les fermentations sur les milieux E et F (jus de diffusion de betterave) et G et H (sirop brut de betterave) donnent des productivités inférieures aux fermentations réalisées sur les milieux A, B et D.

**Tableau 2 : Composition des milieux de culture testés dans l'exemple 2**

| **Ingrédients en g/l** | **A** | **B** | **C** | **D** | **E** | **F** | **G** | **H** |
|---|---|---|---|---|---|---|---|---|
| **Saccharose** | 180 | 0 | 0 | 0 | 0 | 0 | 0 | 0 |
| **Jus vert de canne sec** | 0 | 200 | 0 | 0 | 0 | 0 | 0 | 0 |
| **Sirop brut de canne** | 0 | 0 | 300 | 300 | 0 | 0 | 0 | 0 |
| **Jus de diffusion de betterave sec** | 0 | 0 | 0 | 0 | 200 | 200 | 0 | 0 |
| **Sirop brut de betterave** | 0 | 0 | 0 | 0 | 0 | 0 | 300 | 300 |
| **Yeast extract** | 5 | 0 | 0 | 0 | 0 | 0 | 0 | 0 |
| **K2HPO4** | 0,5 | 0 | 0 | 0,5 | 0 | 0,5 | 0 | 0,5 |
| **KH2PO4** | 0,5 | 0 | 0 | 0,5 | 0 | 0,5 | 0 | 0,5 |
| **MgSO4.7H2O** | 0,2 | 0 | 0 | 0,2 | 0 | 0,2 | 0 | 0,2 |
| **MnCl2.4H2O** | 0,03 | 0 | 0 | 0,03 | 0 | 0,03 | 0 | 0,03 |
| **(NH4)Cl** | 1 | 0 | 0 | 1 | 0 | 1 | 0 | 1 |
| **FeCl3.6H2O** | 0,01 | 0 | 0 | 0,01 | 0 | 0,01 | 0 | 0,01 |

### Exemple 3 : Culture de Bacillus Coagulans sur jus vert de canne sec d'origines géographiques différentes

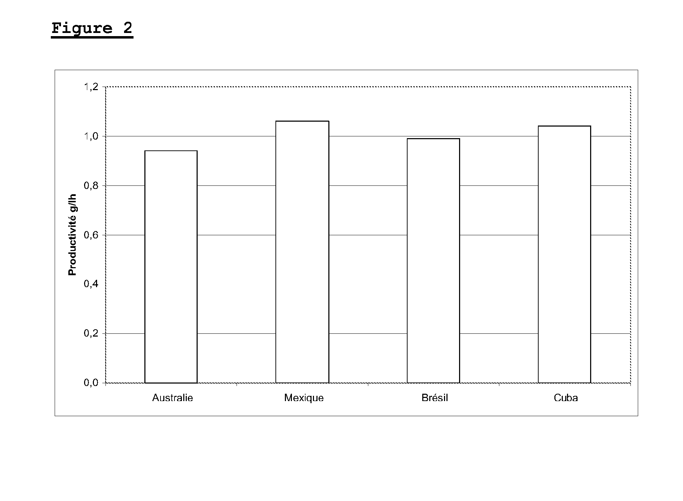

De manière à établir si l'origine géographique du jus vert de canne avait une influence sur la productivité en acide lactique de Bacillus Coagulans (LMG 17452), nous avons cultivé cette bactérie sur un milieu constitué uniquement d'eau et de jus vert de canne sec d'origines diverses (Australie, Mexique, Brésil, Cuba) à 50 g/l à 52°C et maintenu à pH 6,2 avec du lait de Ca(OH)₂ 25% en poids. La culture a été maintenue en routine en transférant 250 ml de la culture toutes les 24 h dans un nouveau fermenteur contenant 750 ml de milieu. La vitesse de production d'acide lactique a été suivie durant 5 fermentations d'affilée (sur la figure 2, chaque colonne est représentative de la moyenne des résultats des 5 essais).

Sur la figure 2, les résultats montrent clairement que les productivités en acide lactique sont semblables (entre 0,9 g/lh et 1,1 g/lh) quelle que soit l'origine géographique de la canne à sucre.

### Exemple 4 : Purification de l'acide lactique produit à partir de sirop de canne brut avec sels

### Fermentation

Bacillus Coagulans (LMG 17452) a été cultivée dans un réacteur BBraun 50 1 sur un milieu de fermentation (identique au milieu D de l'exemple 2 mais une concentration en substrat carboné de 75 g/l au lieu de 300 g/l) constitué de sirop brut de canne (65% en saccharose) avec sels à 52°C et maintenu à pH 6,2 avec un lait de Ca(OH)₂ 25% en poids. Les résultats sont donnés dans le tableau 3.

**Tableau 3 : Résultats de la fermentation**

| **Productivité en acide lactique g/lh** | **Concentration finale en acide lactique g/l** | **Rapport volume final/volume initial (dilution par chaux)** | **Rendement acide lactique produit sur saccharose initial (g/g)** |
|---|---|---|---|
| 1 | 44 | 1,05 | 0, 95 |

Le jus de fermentation obtenu a été purifié selon le procédé décrit ci-dessous.

### Purification basse concentration

Après avoir floculé la biomasse et l'avoir filtrée, le jus clarifié est acidifié par ajout progressif d'acide sulfurique concentré de manière à précipiter le calcium sous forme de CaSO4. Le CaSO4 est alors séparé par filtration.

Le jus est alors pré-purifié sur une colonne de charbon actif. Le percolat est alimenté sur une colonne échangeuse d'ions, remplie d'une résine cationique forte (type Bayer Lewatit S 2528). Une fois la décationisation réalisée, le jus est alimenté sur une colonne remplie d'une résine anionique de basicité moyenne (type Bayer sous la référence Lewatitt S 4328). L'acide lactique obtenu présente alors les caractéristiques suivantes:
- Coloration APHA : 200 Hazen
- Teneur en acide lactique : 45 g/l
- Teneur en cation : <50 meq/l
- Teneur en anion : <50 meq/l

### Purification haute concentration

Le jus purifié ci-dessus est concentré à 80% sur un évaporateur à film ruisselant avant d'être alimenté en continu dans un évaporateur en verre borosilicaté à film mince mécaniquement agité avec condenseur interne. Les paramètres de concentrations sont de 100°C pour les parois et une pression absolue de 100 mbars.

L'acide lactique est enfin distillé à 10 mbars et 130°C sur ce même évaporateur.

L'acide lactique produit par cette purification respecte les caractéristiques (tableau 4) d'un acide lactique stable à la chaleur. En effet, la solution fraîche a une coloration de 11 Hazen et sa coloration après chauffe atteint 15 Hazen.

L'acide lactique produit par cette purification respecte les spécifications d'un acide lactique de haute pureté utilisable pour la fabrication de lactide et d'acide polylactique.

**Tableau 4 : Caractéristiques de l'acide lactique purifié obtenu à partir d'une solution d'acide lactique provenant de la fermentation par Bacillus Coagulans (LMG 17452) d'un milieu composé de sirop de canne brut**

| **Teneur en acide lactique** | % *poids* | 90,5 |
|---|---|---|
| **Pureté stéréochimique L- acide lactique** | *%L*/ *(%L+%D)* | 99,5% |
| **Couleur (solution fraîche)** | *Hazen* | 11 |
| **Couleur après chauffe (200°C, 2h)** | *Hazen* | 15 |

Afin de démontrer l'adéquation de l'acide lactique produit pour la production d'acide polylactique nous avons lancé les essais suivants.

### Synthèse du lactide et test de polymérisation

L'acide lactique obtenu ci-dessus (~250 g) est introduit dans un ballon agité et chauffé à 160°C. Afin de faciliter l'extraction rapide du composé volatile, l'unité est placée progressivement sous vide, la pression variant entre pression atmosphérique et 150 mbars pendant environ 10h. L'acide lactique polymérise pour former un prépolymère caractérisé par une masse moléculaire de 1500 daltons.

Le prépolymère obtenu ci-dessus est introduit dans un ballon chauffé par une calotte chauffante à 220-250°C et agité par une puce magnétique. L'octoate d'étain est alors introduit dans le ballon à 1% en poids par rapport à la quantité de prépolymère introduit.

Le ballon est surmonté d'un reflux à 180-200°C , ensuite d'un condenseur refroidi à 80-100°C et enfin d'un ballon de récolte des condensats. Le tout est mis sous vide entre 10 et 20 mbars. Le lactide impur récolté dans le ballon des condensats est purifié deux fois par recristallisation dans un rapport 1 : 1 avec du toluène.

Les cristaux de lactide purifié sont récupérés par filtration et séchés sous vide dans un rotavapor.

Le lactide purifié de cette manière présente les caractéristiques suivantes :
- L-lactide : 99,8%
- méso-lactide : 0,2%
- acidité résiduelle : <10 meq/kg
- teneur en eau : 49 ppm

Une petite quantité du produit purifié obtenu ci-dessus (10g) a été introduit dans un tube à essai sous balayage d'azote (plusieurs essais ont été lancés en parallèle). Après solubilisation du mélange (100°C) une solution d'octoate d'étain a été ajoutée de sorte à respecter un rapport molaire dimère/catalyseur de 4500. Une fois la solution bien homogénéisée, elle est plongée dans un bain d'huile dont la température est thermostatisée à 180°C.

Après une heure de synthèse, les tubes à essais sont enlevés et cassés de sorte à récupérer des polymères bien rigides et opaques. Les polymères obtenus ont été analysés par GPC dans le chloroforme à 35°C et des masses moléculaire en nombre comprises entre 80.000 et 100.000 ont été mesurées (les masses moléculaires déterminées sur base d'une calibration PS sont corrigées sur une base absolue en utilisant une calibration universelle).

Cet exemple montre donc qu'il est possible d'atteindre un acide lactique de qualité « polymère » en utilisant comme substrat carboné de fermentation du sirop brut de canne.

### Exemple 5 : Purification de l'acide lactique produit à partir de mélasse de canne à sucre

Dans l'exemple 2, nous avons montré que, contrairement aux dérivés de la betterave, les dérivés de la canne permettaient, sous la forme d'un milieu autosuffisant, d'obtenir une fermentation lactique comparable à des milieux industriels classiques. Dans cet exemple, nous allons montrer que certains dérivés de la canne, bien qu'autosuffisant, ne permettent pas de produire un acide lactique de qualité stable à la chaleur.

Bacillus Coagulans (LMG 17452) a été cultivée dans un réacteur BBraun 50 1 sur un milieu de fermentation (identique au milieu D de l'exemple 2 avec une concentration en substrat carboné de 75 g/l au lieu de 300 g/l) constitué de mélasse de canne à sucre (60% en saccharose) avec sels, à 52°C et maintenu à pH 6,2 avec un lait de Ca(OH)₂ 25% en poids. Les résultats sont donnés dans le tableau 5.

**Tableau 5 : Résultats de la fermentation**

| **Productivité en acide lactique g/lh** | **Concentration finale en acide lactique g/l** | **Rapport volume final/volume initial (dilution par chaux)** | **Rendement acide lactique produit sur saccharose initial (g/g)** |
|---|---|---|---|
| 1 | 40 | 1,05 | 0, 94 |

Le jus de fermentation obtenu a été purifié selon la même procédure que l'exemple 4. Le produit suivant a été obtenu après purification basse concentration :
- Coloration APHA : 200 Hazen
- Teneur en acide lactique : 45 g/l
- Teneur en cation : <50 meq/l
- Teneur en anion : <50 meq/l

Par contre, de manière surprenante, l'acide lactique produit par la purification haute concentration ne respecte pas les spécifications d'un acide lactique de haute pureté utilisable pour la fabrication de lactide et d'acide polylactique. En effet, la solution fraîche a déjà une coloration de 70 Hazen et sa coloration après chauffe atteint 230 Hazen.

Il est donc surprenant que dans le cas de l'exemple 4, l'utilisation de sirop de canne brut comme source de carbone et d'azote organique pour la fermentation permette d'obtenir un acide lactique de haute pureté (thermostable).

**Tableau 6 : Caractéristiques de l'acide lactique purifié obtenu à partir d'une solution d'acide lactique provenant de la fermentation par Bacillus Coagulans (LMG 17452) d'un milieu composé de mélasse de canne à sucre**

| | | |
|---|---|---|
| **Teneur en acide lactique** | % *poids* | 90,2 |
| **Couleur (solution fraîche)** | *Hazen* | 70 |
| **Couleur après chauffe (200°C, 2h)** | *Hazen* | 230 |

### Exemple 6: Essai de fermentation de sirop brut de canne avec d'autres micro-organismes

Différentes souches ont été cultivées dans des réacteurs BBraun 2 1 sur un milieu de fermentation (voir exemple 4) constitué de sirop brut de canne (65% en saccharose) avec sels à 52°C et maintenu à pH 6,2 avec un lait de Ca(OH)₂ 25% en poids. Les résultats sont donnés dans le tableau 7.

Dans le tableau 7 ci-dessous nous pouvons voir que le sirop de canne n'est pas un milieu autosuffisant pour tous les micro-organismes producteurs d'acide lactique mais seulement pour quelques uns dont certaines espèces de Bacillus et Sporolactobacillus.

**Tableau 7 : Fermentation du sirop brut de canne au départ de micro-organismes producteurs d'acide lactique**

| | **Origine** | **Productivité (g/lh)** | **Pureté stéréochimique (% en L)** | **Acide lactique final produit (g)** |
|---|---|---|---|---|
| **Bacillus Coagulans** | LMG 17452 | 1 | 99 | 45 |
| **Bacillus Coagulans** | LMG 19808 | 1 | 99 | 40 |
| **Sporolactobacillus Inulinus** | DSM20348 | 0,5 | 1 | 20 |
| **Bacillus Smithii** | DSM 459 | 0,5 | 97 | 20 |
| **Lactobacillus Plantarum** | DSM 2601 | 0 | | 0 |
| **Lactobacillus Coryniformis** | DSM 20004 | 0 | | 0 |

### Exemple 7 : Amélioration de la fermentation en enrichissant le milieu de fermentation avec de l'extrait de levure

Comme décrit dans les exemples ci-dessus, un milieu de fermentation autosuffisant préparé à partir de jus vert de canne et fermenté avec Bacillus Coagulans (LMG 17452) permet une productivité en acide lactique comparable à des milieux complexes préparé à partir de sucres purifiés, de minéraux et d'une source d'azote organique.

Nous avons observé que cette productivité pouvait encore être améliorée (+20%) en ajoutant une source d'azote organique exogène (extrait de levure à 0,5 g/l) à ce milieu préparé à partir de jus vert de canne.

L'invention concerne un procédé de production d'acide lactique par fermentation d'un extrait de canne à sucre ou de dérivés d'extraits de canne à sucre au moyen de micro-organismes. Ce procédé est caractérisé par les micro-organismes de fermentation qui appartiennent aux genres Bacillus, Sporolactobacillus ou leurs mélanges, et par le milieu de fermentation qui est autosuffisant.

Ce procédé comporte de façon préférée au moins une étape de purification de l'acide lactique issu de la fermentation. La purification comporte de préférence au moins une étape choisie parmi l'évaporation, la distillation, la cristallisation ou l'utilisation de résines échangeuses d'ions.

Les micro-organismes sont automatiquement choisis parmi les espèces Bacillus Coagulans, Bacillus Smithii, Sporolactobacillus Inulinus ou leurs mélanges.

Le procédé produit un acide lactique stable à la chaleur.

La canne à sucre ou le dérivé de la canne à sucre a avantageusement une concentration en azote organique supérieure à 0,02 g/kg de milieu de fermentation. L'extrait de la canne à sucre est de préférence choisi parmi le jus vert de canne, le sirop brut de canne, le sirop brut de canne inversé, le sucre de canne brut ou leurs dérivés. L'extrait de canne à sucre ou les dérivés d'extraits de canne à sucre se présentent de préférence sous forme liquide ou sous forme de solide sec.

Le dérivé d'extrait de canne à sucre est obtenu par une méthode de concentration avantageusement choisie parmi l'atomisation, l'évaporation, la cristallisation ou la centrifugation.

Le milieu de fermentation est, dans un mode de réalisation, enrichi par des autolysats et hydrolysats de levure, des hydrolysats de protéines végétales ou animales, ou par des sous-produits solubles de trempe de blé ou de maïs.

Le milieu est, dans un mode de réalisation, enrichi avec un sucre purifié choisi parmi le glucose, le maltose, le fructose, le xylose ou le saccharose.

L'extrait de canne à sucre ou les dérivés d'extraits de canne à sucre est, dans un mode de réalisation, stérilisé de manière mécanique, thermique ou chimique avant fermentation.

## Revendications

1. Procédé de production d'acide lactique par fermentation d'un extrait de canne à sucre ou de dérivés d'extraits de canne à sucre au moyen de micro-organismes, **caractérisé en ce que** :
a) les micro-organismes de la fermentation appartiennent aux genres Bacillus, Sporolactobacillus ou leurs mélanges ;
b) le milieu de fermentation est autosuffisant.

2. Procédé selon la revendication 1, **caractérisé en ce qu'**il comporte au moins une étape de purification de l'acide lactique issu de la fermentation.

3. Procédé selon l'une quelconque des revendications précédentes, **caractérisé en ce que** la purification comporte au moins une étape choisie parmi l'évaporation, la distillation, la cristallisation ou l'utilisation de résines échangeuses d'ions.

4. Procédé selon l'une quelconque des revendications précédentes, **caractérisé en ce que** les micro-organismes sont choisis parmi les espèces Bacillus Coagulans, Bacillus Smithii, Sporolactobacillus Inulinus ou leurs mélanges.

5. Procédé selon l'une quelconque des revendications précédentes, **caractérisé en ce que** l'acide lactique produit est stable à la chaleur.

6. Procédé selon l'une quelconque des revendications précédentes, **caractérisé en ce que** la canne à sucre ou le dérivé de la canne à sucre a une concentration en azote organique supérieure à 0,02 g/kg de milieu de fermentation.

7. Procédé selon l'une quelconque des revendications précédentes, **caractérisé en ce que** l'extrait de la canne à sucre est choisi parmi le jus vert de canne, le sirop brut de canne, le sirop brut de canne inversé, le sucre de canne brut ou leurs dérivés.

8. Procédé selon l'une quelconque des revendications précédentes, **caractérisé en ce que** l'extrait de canne à sucre ou les dérivés d'extraits de canne à sucre se présentent sous forme liquide ou sous forme de solide sec.

9. Procédé selon l'une quelconque des revendications précédentes, **caractérisé en ce que** le dérivé d'extrait de canne à sucre est obtenu par une méthode de concentration choisie parmi l'atomisation, l'évaporation, la cristallisation ou la centrifugation.

10. Procédé selon l'une quelconque des revendications précédentes, **caractérisé en ce que** le milieu est enrichi par des autolysats et hydrolysats de levure, des hydrolysats de protéines végétales ou animales, ou par des sous-produits solubles de trempe de blé ou de maïs.

11. Procédé selon l'une quelconque des revendications précédentes, **caractérisé en ce que** le milieu est enrichi avec un sucre purifié choisi parmi le glucose, le maltose, le fructose, le xylose ou le saccharose.

12. Procédé selon l'une quelconque des revendications précédentes, **caractérisé en ce que** l'extrait de canne à sucre ou les dérivés d'extraits de canne à sucre est stérilisé de manière mécanique, thermique ou chimique avant fermentation.
